# EUROPÄISCHE PATENTANMELDUNG

(11) **EP 3 228 336 A1**
(43) Veröffentlichungstag der Anmeldung: **11.10.2017**
(21) Anmeldenummer: 16164527.0
(22) Anmeldetag: 08.04.2016
(51) Int. Cl.: A61M 1/10

(54) **KANÜLENANORDNUNG UND BLUTPUMPENANORDNUNG SOWIE DEREN VERWENDUNG**

(71) Anmelder: Berlin Heart GmbH, 12247 Berlin (DE)
(72) Erfinder: ARSLAN, Nedim, 10717 Berlin (DE); WINTERWERBER, Kim Peter, 12357 Berlin (DE); MATTHES, Michael, 15345 Altlandsberg (DE); GUNDLACH, Heiko, 10435 Berlin (DE); KAEBE, Benjamin Daniel, Scullin ACT, 2614 (AU); LAUTERBACH, Gerhard, 12679 Berlin (DE)
(74) Vertreter: Pfenning, Meinig & Partner mbB

(57) **Zusammenfassung**

Die Erfindung betrifft eine Kanülenanordnung (2), umfassend eine Kanüle (13), insbesondere einen Graft, zur Bildung eines Fließkanals (16) für Körperflüssigkeiten, insbesondere für Blut, und ein Schutzelement (14) für die Kanüle (13), wobei das Schutzelement (14) einen Kanal (15) für die Kanüle (13) definiert, wobei die Kanüle (13) zumindest abschnittsweise durch den Kanal (15) des Schutzelements (14) hindurch verläuft, wobei das Schutzelement (14) eine Mehrzahl von Segmenten (17) umfasst, wobei die Segmente (17) in einer Abfolge nebeneinander angeordnet sind und wobei jedes der Segmente (17) einen Teilabschnitt des Kanals (15) des Schutzelements (14) definiert. Die Erfindung betrifft außerdem eine Blutpumpenanordnung (1) und ein Verfahren zum Verwenden der Kanülenanordnung (2) und der Blutpumpenanordnung (1).

## Beschreibung

Die Erfindung liegt auf dem Gebiet der Medizintechnik und betrifft eine Kanülenanordnung und eine Blutpumpenanordnung sowie deren Verwendung.

Unter einer Kanüle wird im vorliegenden Zusammenhang ein Leitungselement verstanden, das geeignet ist, einen Fließkanal für eine körpereigene Flüssigkeit, wie beispielsweise Blut, zu bilden. Von besonderem Interesse sind implantierbare Kanülen, welche vorübergehend oder dauerhaft in körpereigenes Gewebe eines Patienten eingebettet werden können. Hierzu bestehen die Kanülen in der Regel ganz oder zumindest teilweise, insbesondere auf ihren Oberflächen, aus einem biokompatiblen Material. Ein Beispiel für eine implantierbare Kanüle ist ein Graft, der im Allgemeinen eine textile Trägerstruktur aufweist. Diese kann bereits vor ihrer Verwendung mit einem dichtenden Material, wie etwa Rindergelatine, abgedichtet sein oder aber auch erst durch die Tränkung mit Blut abgedichtet werden (sogenanntes Glotting).

Grafts werden beispielsweise als Gefäßprothese verwendet, wobei sie mit einem Ende mit einem Blutgefäß oder dem Herzen des Patienten verbunden werden, beispielsweise durch direktes Vernähen oder mittels eines Nahtrings, und mit dem anderen Ende beispielsweise direkt oder indirekt mit einem weiteren Leitungselement und/oder einer implantierten oder externen Blutpumpe.

Implantierbare Kanülen, insbesondere Grafts, sind typischerweise sehr flexibel und leicht verformbar. Diese durchaus gewollten Eigenschaften von Grafts und vergleichbaren flexiblen Kanülen können, insbesondere bei der Implantation, mitunter auch zu Problemen führen. Beispielsweise besteht die Gefahr, dass die Kanüle geknickt oder zusammengedrückt wird. Außerdem besteht das Risiko, dass die Kanüle bei der Implantation oder bei einem nachfolgenden operativen Eingriff beschädigt wird, beispielsweise durch ein Skalpell oder ein anderes Instrument.

Es stellt sich daher die Aufgabe, die oben beschriebenen Probleme ganz oder zumindest teilweise zu lösen und möglichst auch die Handhabung einer Kanüle zu vereinfachen. Eine verbesserte Kanülenanordnung sollte also einen möglichst guten mechanischen Schutz für eine Kanüle der Kanülenanordnung bieten. Außerdem sollte sich eine verbesserte Kanülenanordnung durch eine möglichst einfache Handhabung auszeichnen, insbesondere vor oder während der Implantation. Eine entsprechend verbesserte Blutpumpennordnung sowie ein entsprechend verbessertes Verfahren zum Verwenden der Kanülenanordnung und der Blutpumpenanordnung sind ebenfalls wünschenswert.

Die oben beschriebene Aufgabe wird durch eine Kanülenanordnung gemäß dem Hauptanspruch sowie durch eine Blutpumpenanordnung und eine Verwendung gemäß den nebengeordneten Ansprüchen gelöst. Weiterentwicklungen ergeben sich mit den jeweils abhängigen Ansprüchen sowie mit der nachfolgenden Beschreibung und den Figuren.

Eine Kanülenanordnung hier vorgeschlagener Art umfasst daher eine Kanüle der eingangs beschriebenen Art zur Bildung eines Fließkanals für Körperflüssigkeiten, insbesondere für Blut. Im Fall, dass die Kanüle ein Graft ist, umfasst die Kanüle typischerweise eine textile Trägerstruktur, welche beispielsweise schlauchförmig oder wellschlauchförmig ausgestaltet sein kann. Die textile Trägerstruktur ist beispielsweise aus Fasern gebildet. Als Fasern kommen insbesondere Kunststofffasern in Frage, wie etwa Polyesterfasern. Die Fasern der Trägerstruktur können beispielsweise gewebt oder gestrickt sein. Die Trägerstruktur kann mit Dichtungsmaterial abgedichtet und somit für die zu leitende Flüssigkeit undurchlässig sein, beispielsweise mit Gelatine, wie etwa Rindergelatine. Die Kanüle kann an einem oder an beiden ihrer Enden Anschlusselemente aufweisen, wie bereits oben erwähnt worden ist und weiter unten noch detaillierter beschrieben wird.

Die Kanülenanordnung umfasst zusätzlich zur Kanüle und als ein von der Kanüle unabhängig hergestelltes, separates Bauteil der Kanülenanordnung ein ebenfalls implantierbares Schutzelement für die Kanüle. Das Schutzelement hat die Funktion, Beschädigungen der Kanüle aufgrund von mechanischen Belastungen, die von außen auf die Kanüle einwirken, möglichst zu verhindern oder zu minimieren. Insbesondere reduziert das Schutzelement das Risiko, dass die Kanüle geknickt oder zusammengedrückt wird oder durch ein Skalpell oder ein anderes Instrument beschädigt wird. Zu diesem Zweck definiert bzw. umgrenzt das Schutzelement einen Kanal für die Kanüle. Die Kanüle verläuft vollständig oder zumindest abschnittsweise durch diesen Kanal hindurch. Entsprechend verläuft auch der durch die Kanüle umgrenzte Fließkanal vollständig oder zumindest abschnittsweise innerhalb des Kanals des Schutzelements. Die Kanüle wird durch den Kanal des Schutzelements somit seitlich geführt. Daher wird der Kanal des Schutzelements im Folgenden auch als Führungskanal bezeichnet. Der Führungskanal und der Fließkanal der Kanüle verlaufen, zumindest in den Abschnitten, in denen der Fließkanal durch den Führungskanal verläuft, im Wesentlichen parallel und koaxial zueinander.

Das hier vorgeschlagene Schutzelement umfasst eine Mehrzahl von Segmenten, beispielsweise also zwei oder mehr Segmente, vorzugsweise mehr als drei oder mehr als vier Segmente, typischerweise zwischen 4 und 20 Segmente. Die Segmente sind in einer Abfolge nebeneinander angeordnet. Jedes der Segmente definiert einen axialen Teilabschnitt des Kanals des Schutzelements, wobei der Teilabschnitt das jeweilige Segment vollständig durchläuft. Zu diesem Zweck sind die Segmente beispielsweise hülsenförmig ausgeformt und/oder weisen jeweils eine Durchtrittsöffnung auf, welche das jeweilige Segment vollständig in Richtung der Abfolge der Segmente durchläuft und jeweils einen Teilabschnitt des Kanals bildet.

Beim Zusammenbau der Kanülenanordnung wird die Kanüle ganz oder zumindest abschnittsweise in den Kanal des Schutzelements eingebracht, typischerweise indem die Kanüle in den Kanal gezogen oder (eine ausreichende Stabilität der Kanüle vorausgesetzt) geschoben wird oder indem das Schutzelement über die Kanüle geschoben oder gezogen wird. Der Führungskanal ist vorzugsweise ausreichend breit, um axiale Verschiebungen des Schutzelements relativ zu der im Führungskanal verlaufenden Kanüle wie auch axiale Ausgleichsbewegungen zwischen dem Schutzelement und der Kanüle (insbesondere bei Biegebewegungen der Kanülenanordnung) zu ermöglichen. Hierdurch können insbesondere auch auf die Kanüle einwirkende Zugbelastungen minimiert werden, welche insbesondere im Fall empfindlicher Grafts zu Beschädigungen führen können. Zu diesem Zweck kann beispielsweise ein (kleinster) Durchmesser oder eine (kleinste) Querschnittsfläche des Führungskanals größer sein als ein (größter) Außendurchmesser bzw. eine (größte) äußere Querschnittsfläche der Kanüle.

Entsprechend kann beispielsweise ein (kleinster) Innendurchmesser der Segmente, also der (kleinste) Durchmesser der oben genannten Durchtrittsöffnungen der Segmente, größer sein als ein (größter) Außendurchmesser der Kanüle. Auf diese Weise kann zwischen Innenwänden der Segmente, die den Führungskanal jeweils radial umgrenzen (und die jeweiligen Durchtrittsöffnungen definieren), und einer äußeren Oberfläche der Kanüle ein freier Zwischenspalt bestehen, der ganz oder zumindest abschnittsweise in Form eines Ringspalts ausgestaltet sein kann.

Die Segmente des Schutzelements sind miteinander verbunden. Das Schutzelement bildet somit eine Einheit, die unabhängig von der Kanüle oder anderen Bauteilen mechanisch zusammenhält.

Zur Verbesserung der Handhabung der Kanülenanordnung können die Schutzelemente beispielsweise derart miteinander verbunden sein, dass relative Drehbewegungen der Segmente relativ zueinander um eine Längsachse des Kanals (bzw. des Schutzelements) möglich sind, ohne hierbei die Verbindungen zwischen den Segmenten zu lösen oder zu beschädigen. Zum Beispiel sind die Segmente derart miteinander verbunden, dass in der Abfolge benachbarte Segmente relativ zueinander um eine Längsachse des Kanals um mindestens 3° oder um mindestens 5° drehbar sind, ohne die Verbindung zwischen diesen Segmenten zu lösen oder zu beschädigen. Es ist auch m öglich, dass die Segmente derart miteinander verbunden sind, dass in der Abfolge benachbarte Segmente relativ zueinander um eine Längsachse des Kanals um 360° drehbar sind, ohne die Verbindung zwischen diesen Segmenten zu lösen oder zu beschädigen. Durch die beschriebenen Drehungen ist es beispielsweise auch möglich, dass einzelne oder mehrere Segmente des Schutzelements relativ zu der Kanüle um die Längsachse der Kanüle gedreht werden. Im Folgenden werden verschiedene Beispiele für Verbindungen gegeben, welche die beschriebenen Drehbewegungen zwischen den Segmenten erlauben.

Beispielsweise kann das Schutzelement Verbindungselemente umfassen, welche die Segmente miteinander verbinden. Die Verbindungselemente können beispielsweise stoffschlüssig mit den Segmenten verbunden sein, beispielsweise mittels eines Klebstoffs. Beispielsweise kann jedes der Verbindungselemente mit zwei oder mit mehr als zwei der Segmente oder mit jedem der Segmente stoffschlüssig verbunden sein, beispielsweise durch Verkleben des Verbindungselements mit den jeweiligen Segmenten. Die Verbindungselemente können getrennt von den Segmenten hergestellte Bauteile sein oder aber integrale Bestandteile der Segmente bzw. Bereiche der Segmente sein. Typischerweise befinden sich die Verbindungselemente radial außerhalb des Führungskanals. Beispiele für die Verbindungselemente werden weiter unten angegeben.

Die Segmente können beispielsweise formschlüssig miteinander verbunden sein. Dies kann beispielsweise mittels der genannten Verbindungselemente erfolgen, welche in die Segmente integriert sein können und/oder Teile oder Teilbereiche der Segmente sein können, wie weiter unten näher beschrieben wird.

Um dem Schutzelement Flexibilität zu verleihen und gleichzeitig einen einfachen und relativ kostengünstigen Aufbau zu ermöglichen, können die Segmente ganz oder zumindest teilweise aus einem elastisch verformbaren Polymer gebildet sein. Zusätzlich oder alternativ hierzu können auch die Verbindungselemente ganz oder zumindest teilweise aus einem elastisch verformbaren Polymer gebildet sein. Das Polymer der Segmente und das Polymer der Verbindungselemente können identisch oder voneinander verschieden sein. Das jeweilige Polymer kann genau ein Polymer oder eine Mischung aus mehreren verschiedenen Polymeren sein. Beispielsweise können die Segmente und/oder die Verbindungselemente ganz oder zumindest teilweise aus einem Silikon, einem Silikonelastomer, einem Polyurethan oder einem anderen elastisch verformbaren Kunststoff gebildet sein. Beispielsweise ist es aber auch möglich, dass die Segmente nicht ganz oder lediglich teilweise aus einem elastisch verformbaren Polymer gebildet sind, sondern beispielsweise aus einem formstabilen Polymer oder aus einem Metall, wie beispielsweise Titan. Die Flexibilität des Schutzelements kann in diesem Fall hauptsächlich durch die Verbindungselemente erreicht werden, insbesondere wenn diese ganz oder teilweise aus einem formflexiblen Material, wie dem genannten Polymer, gebildet sind oder wenn sie aufgrund ihrer Form formflexibel oder elastisch verformbar sind. Geeignete Formen sind beispielsweise kabelartige, drahtartige oder schlauchartige Formen mit ausreichend kleinen Außendurchmessern.

In dem elastisch verformbaren Polymer der Segmente und/oder der Verbindungselemente können weitere Materialen integriert sein, wie beispielsweise Metalle, insbesondere Titan. Die Segmente und/oder die Verbindungselemente können also ganz oder teilweise aus Verbundwerkstoffen bestehen, wobei das jeweilige elastisch verformbare Polymer des Segments bzw. des Verbindungselements typischerweise die Matrix des jeweiligen Verbundwerkstoffs darstellt.

Die Segmente und/oder die Verbindungselemente können beispielsweise Formteile sein. Beispielsweise können die Segmente und/oder die Verbindungselemente Spritzgussteile sein, also durch Spritzgießen hergestellt worden sein. Die Segmente können in einem mechanisch unbelasteten Zustand formidentisch sein. Ebenso können die Verbindungselemente in einem mechanisch unbelasteten Zustand formidentisch sein. Im Fall von Spritzgussteilen kann somit jeweils eine Spritzgussform für die Segmente bzw. die Verbindungselemente ausreichend sein.

In einer speziellen Ausführungsform der Kanülenanordnung umfassen die Verbindungselemente des Schutzelements mindestens ein kabelförmiges Element. Unter einem kabelförmigen Element werden sich entlang der Längsachse erstreckende Elemente, wie beispielsweise Kabel, verstanden, die sich über mehrere Segmente erstrecken. Dabei ist die Erstreckung entlang der Dimension der Längsachse deutlich größer als in den verbleibenden Dimensionen quer zur Längsachse. Neben kabelförmigen Elementen, welche einen kabelartigen Querschnitt aufweisen, können die kabelförmigen Elemente auch einen bandartigen Querschnitt (daher hier auch als bänderförmige Elemente bezeichnet) aufweisen, d.h., das kabelförmige Element kann quer zur Längsrichtung deutlich breiter als hoch sein. Dabei kann die Breite mehr als 10-mal so groß sein wie die Höhe. Die Breite ist jedoch geringer als die Länge. Die bänderförmigen Elemente können in einer Ausführungsform auch schlauchförmige Elemente sein, welche die Segmente in ihrem gesamten Umfang einhüllen. Hierbei muss jedoch eine hohe Flexibilität des Materials des schlauchförmigen Elements gewährleistet sein, damit die Segmente gegeneinander verdreht oder geneigt werden können. In einigen Ausführungsformen der kabelförmigen Elemente ist es möglich, dass jedes der Verbindungselemente durch ein solches kabelförmiges Element gegeben ist oder dass das Schutzelement neben den kabelförmigen Elementen noch andere Verbindungselemente umfasst. Das mindestens eine Verbindungselement, beispielsweise in Form eines kabelförmigen Elements, ist beispielsweise mit zwei oder mit mehr als zwei der Segmente oder mit jedem der Segmente direkt verbunden und an diesen direkt befestigt, beispielsweise durch Verkleben. Das jeweilige Verbindungselement bzw. kabelförmige Element erstreckt sich in einer axialen Richtung, beispielsweise also entlang des Führungskanals bzw. entlang einer Längsachse der Kanülenanordnung, entlang aller Segmente oder zumindest entlang derjenigen Segmente, mit denen das Verbindungselement bzw. kabelförmige Element direkt verbunden ist. Das Verbindungselement, beispielsweise in Form eines kabelförmigen Elements, oder die Verbindungselemente, beispielsweise in Form kabelförmiger Elemente, kann bzw. können beispielsweise schlauchartig oder drahtartig sein.

Verbindungen zwischen den Segmenten mittels Verbindungselementen in Form derartiger kabelförmiger Elemente erlauben es beispielsweise, die Segmente derart miteinander zu verbinden, dass die oben beschriebenen Drehbewegungen zwischen den Segmenten möglich sind. Eine Verdrehung von 360° oder mehr, bevorzugt von 270° oder mehr bzw. von 180° oder mehr, ist in einigen Ausführungsbeispielen ausgeschlossen.

Falls zwei oder mehr Verbindungselemente, wie beispielsweise die genannten kabelförmigen Elemente, vorgesehen sind, können diese bezüglich einer (azimutalen) Umfangsrichtung um das Schutzelement herum bzw. um die Segmente herum voneinander beabstandet angeordnet sein und vorzugsweise gleichmäßig über den Umfang der Segmente verteilt sein. Sind beispielsweise genau drei (vier) Verbindungselemente (z.B. kabelförmige Elemente) vorhanden, haben diese vorzugsweise einen gleichmäßigen gegenseitigen Abstand in Umfangsrichtung der Segmente von beispielsweise 120° (90°).

Die Segmente können radial nach außen gerichtete Vorsprünge aufweisen, an denen das mindestens eine Verbindungselement, beispielsweise in Form eines kabelförmigen Elements, abgestützt und/oder befestigt ist (zum Beispiel durch Kleben). Zwischen den radial nach außen gerichteten Vorsprüngen der Segmente kann das jeweilige Verbindungselement bzw. kabelförmige Element ganz oder zumindest abschnittsweise freitragend verlaufen, also von dem jeweiligen Segment radial beabstandet. Beispielsweise kann dies erreicht werden, indem die axiale Ausdehnung der Vorsprünge geringer als eine axiale Ausdehnung des jeweiligen Segments ist. Hierdurch wird ein Durchtrennen des Verbindungselements bzw. des kabelförmigen Elements, beispielsweise mittels eines handgeführten Schneidinstruments, wie ein Skalpell, erleichtert. Die Vorsprünge können beispielsweise als ringförmige Verbreiterungen der jeweiligen Segmente ausgeformt sein. Die Vorsprünge können beispielsweise Aufnahmen aufweisen, beispielsweise in Form von Vertiefungen, durch die jeweils ein axialer Teilabschnitt eines Verbindungselements bzw. kabelförmigen Elements hindurch verläuft.

Zusätzlich oder alternativ zu den kabelförmigen Elementen kann das Schutzelement auch Verbindungselemente aufweisen, die in axialen Endbereichen der Segmente integriert bzw. durch die Form dieser Endbereiche realisiert sind. Diese Verbindungselemente können beispielsweise ausgestaltet sein, formschlüssige Verbindungen zwischen benachbarten Segmenten herzustellen. Beispielsweise kann jedes der Segmente einen ersten axialen Endbereich und einen zweiten axialen Endbereich aufweisen, wobei die ersten axialen Endbereiche der Segmente komplementär zu den zweiten axialen Endbereichen der Segmente geformt sind. Somit stellen diese Endbereiche Verbindungselemente des Schutzelements dar oder sind Träger für Verbindungselemente des Schutzelements, wie beispielsweise die unten genannten Stege und Nuten. Die ersten und die zweiten axialen Endbereiche von Segmenten, die in der Abfolge benachbart sind, können sich axial überlappen und sich in einem gegenseitigen Eingriff befinden. Durch diesen Eingriff besteht eine mechanische Verbindung zwischen den Segmenten. Vorzugsweise sind die Segmente in diesen axialen Endbereichen elastisch verformbar, beispielsweise weil sie, wie oben beschrieben, aus einem elastisch verformbaren Material bzw. Polymer geformt sind, so dass sich diese Verbindungen manuell durch axiales Zusammenschieben oder Auseinanderziehen jeweils benachbarter Segmente herstellen bzw. lösen lassen.

Beispielsweise kann der erste axiale Endbereich eines jeden Segments einen ersten Steg sowie eine erste Nut aufweisen, wobei der erste Steg und die erste Nut auf einer dem Kanal (radial) abgewandten Außenseite (also auf einer äußeren Oberfläche) des jeweiligen Segments angeordnet sind, wobei der zweite axiale Endbereich eines jeden Segments einen zweiten Steg, der zur ersten Nut komplementär geformt ist, sowie eine zweite Nut, die zum ersten Steg komplementär geformt ist, aufweist, wobei der zweite Steg und die zweite Nut auf einer dem Kanal (radial) zugewandten Innenseite (also einer inneren Oberfläche) des jeweiligen Segments angeordnet sind. Die ersten und zweiten Stege wie auch die ersten und zweiten Nuten können den Kanal jeweils vollständig oder teilweise (azimutal) umlaufen.

Mittels formschlüssiger Verbindungen zwischen den Segmenten ist es insbesondere möglich, die Segmente derart miteinander zu verbinden, dass die oben beschriebenen Drehbewegungen zwischen den Segmenten möglich sind. Außerdem können formschlüssige Verbindungen derart ausgestaltet sein, dass sie sich manuell durch axiales Zusammenschieben oder Auseinanderziehen jeweils benachbarter Segmente herstellen bzw. lösen lassen, wie oben anhand eines konkreten Beispiels beschrieben worden ist.

Beispielsweise können eines, mehrere oder jedes der Segmente des Schutzelements jeweils einen Schlitz aufweisen, der sich in axialer Richtung über eine axiale Gesamtlänge des jeweiligen Segments und in radialer Richtung von einer dem Kanal (radial) abgewandten Außenseite (äußeren Oberfläche) des jeweiligen Segments bis zu einer dem Kanal (radial) zugewandten Innenseite (innere Oberfläche) hin erstreckt. Ein solcher Schlitz kann beispielsweise manuell oder mittels eines geeigneten Instruments durch entsprechendes Verformen des jeweiligen Segments aufgeweitet oder aufgedehnt werden. Wird der Spalt ausreichend weit aufgeweitet bzw. aufgedehnt, kann das jeweilige Segment, sofern es zuvor von den benachbarten Segmenten getrennt worden ist, von der Kanüle in einer seitlichen, nichtaxialen Richtung abgezogen werden. Dies erlaubt eine Entfernung eines geschlitzten Segments also auch dann, wenn die Kanüle bereits mit beiden Enden mit weiteren Leitungselementen und/oder mit Blutgefäßen verbunden ist.

Typischerweise umfasst die Kanülenanordnung ein erstes Ende, im Folgenden auch als hinteres Ende der Kanülenanordnung bezeichnet, das zur mittelbaren oder unmittelbaren Verbindung mit einem Körperorgan oder einem Körperflüssigkeit führenden, insbesondere körpereigenen Hohlraum ausgebildet ist, und ein zweites Ende, im Folgenden auch als vorderes Ende bezeichnet, das zur Verbindung/Konnektierung mit einem Leitungselement (z.B. Auslasskrümmer) oder einem Funktionselement der Flüssigkeitsförderung (Blutpumpe) ausgebildet ist.

Die Kanüle kann mindestens ein Anschlusselement aufweisen. Die Kanüle kann beispielsweise an einem axialen Endbereich der Kanüle sowie bei Bedarf auch an einem hierzu entgegengesetzten weiteren axialen Endbereich der Kanüle jeweils ein Anschlusselement aufweisen.

Mindestens ein Anschlusselement der Kanüle kann dazu ausgestaltet sein, eine Verbindung zwischen der Kanüle und einem körpereigenen Blutgefäß oder Herzen eines Patienten herzustellen. Beispielsweise kann ein solches Anschlusselement ein Nahtring sein.

Mindestens ein Anschlusselement der Kanüle kann dazu ausgestaltet sein, eine fluiddichte Verbindung zwischen der Kanüle und einem weiteren Leitungselement für die zu leitende Flüssigkeit, beispielsweise mit einem in den axialen Endbereich der Kanüle eingeschobenen Rohrelement, herzustellen. Dieses Rohrelement kann beispielsweise ein Teilabschnitt eines Auslasses oder eines Auslasskrümmers einer Blutpumpe oder eines Einlasses oder eines Einlasskrümmers einer Blutpumpe oder auch eine Anschlusskanüle sein. Ein derartiges Anschlusselement kann beispielsweise in Form einer Schelle ausgestaltet sein, welche den jeweiligen axialen Endbereich der Kanüle umgreift. Die Schelle kann in einen gespannten Zustand überführt werden, um eine radial nach innen auf das Endstück der Kanüle einwirkende Klemmkraft auszuüben und somit eine fluiddichte Klemmverbindung zwischen der Kanüle und einem in den axialen Endbereich der Kanüle eingeschobenen Rohrelement herzustellen. Beispielweise kann die Schelle zur Herstellung und/oder zum Stabilisieren des gespannten Zustands der Schelle einen Schraubverschluss mit einer Spannschraube und einem korrespondierenden Gewindeteil oder einen Rastverschluss mit entsprechenden Rastelementen oder ein elastisches Federelement umfassen. Beispielsweise kann die Schelle aus einem Metall und/oder einem Polymer gefertigt sein.

Eine Blutpumpenanordnung hier vorgeschlagener Art umfasst vorzugsweise eine Kanülenanordnung hier vorgeschlagener Art und eine Blutpumpe (VAD), insbesondere eine implantierbare Blutpumpe oder eine externe Blutpumpe. Bei der Blutpumpe kann es sich beispielsweise um eine Axialpumpe oder um eine Zentrifugalpumpe oder um eine pulsatil fördernde Pumpe handeln. Typischerweise umfasst die Blutpumpe einen Einlass und einen Auslass. Im Fall einer linksventrikulären Blutpumpenanordnung kann beispielsweise vorgesehen sein, dass der Einlass der Blutpumpe direkt oder indirekt, beispielsweise über einen Einlasskrümmer, mit einer linken Herzkammer verbunden wird. Der Auslass der Blutpumpe kann direkt oder indirekt, beispielsweise über einen Auslasskrümmer, mit dem vorderen Ende der Kanülenanordnung verbunden sein. Mit dem hinteren Ende kann die Kanüle der Kanülenanordnung mit einem Blutgefäß des Patienten, beispielsweise mit der Aorta bzw. dem Aortenbogen, verbunden werden.

Die Kanülenanordnung kann an dem vorderen Ende der Kanülenanordnung einen Konnektor aufweisen. Beispielsweise kann der Konnektor an dem Schutzelement befestigt sein, beispielsweise an einem vordersten Segment des Schutzelements. Die Blutpumpe kann ein mit dem Konnektor korrespondierendes Verbindungsteil zum Herstellen einer lösbaren Verbindung zwischen der Kanülenanordnung und der Blutpumpe aufweisen. Der Konnektor kann beispielsweise ein Schnappverbinder mit mindestens einem flexiblen Schnappelement sein. Zum Beispiel kann der Konnektor einen Klauenring umfassen, beispielsweise einen Klauenring mit Klauen der in der Veröffentlichung WO 2004/001272 A1 beschriebenen Art. Beispielsweise kann der Klauenring drehbar und/oder verschiebbar auf einem vordersten Ende des Schutzelements, beispielsweise auf dem vordersten Segment des Schutzelements, angeordnet sein.

Das korrespondierende Verbindungsteil kann beispielsweise mindestens eine mit dem mindestens einen flexiblen Schnappelement korrespondierende Haltefläche zum Herstellen einer lösbaren Rastverbindung zwischen der mindestens einen Haltefläche und dem mindestens einen Schnappelement aufweisen. Zum Beispiel kann das Verbindungsteil einen Rastring mit Rastringhalteflächen umfassen, beispielsweise einen Rastring der in der Veröffentlichung WO 2004/001272 A1 beschriebenen Art. Der Rastring kann beispielsweise in das Verbindungsteil integriert sein und beispielsweise als ein axialer Endbereich des Verbindungsteils ausgestaltet sein.

Das mit dem Konnektor der Kanülenanordnung korrespondierende Verbindungsteil der Blutpumpe kann ein Adapterelement umfassen oder als ein Adapterelement ausgestaltet sein. Das Adapterelement kann mit einem Leitungselement der Blutpumpe, wie beispielsweise einem Auslass oder einem Auslasskrümmer der Blutpumpe, fest verbunden sein, beispielsweise mittels eines Klebstoffs, wie etwa ein Silikonkleber. Das Adapterelement kann in einigen Ausführungsbeispielen teilweise oder ganz aus einem metallischen Werkstoff gefertigt ist, beispielsweise aus Titan. Ebenso kann das genannte Leitungselements aus einem metallischen Werkstoff, wie beispielsweise Titan, gefertigt sein oder aber aus einem Polymer, wie etwa aus einem Silikon.

Das Adapterelement kann einen hülsenförmigen Bereich aufweisen, der einen axialen Endbereich des Leitungselements der Blutpumpe vollständig oder teilweise umschließt. Beispielsweise kann der oben genannte Rastring axial an den hülsenförmigen Bereich angrenzen. Der hülsenförmige Bereich kann im Wesentlichen radial verlaufende Durchtrittsöffnungen aufweisen. Ausgehend von dem Leitungselement können dann Befestigungselemente durch die Durchtrittsöffnungen hindurch verlaufen, welche das Adapterelement mit dem Leitungselement verbinden. Beispielsweise sind die Befestigungselemente mit dem Leitungselement mittels eines Klebstoffs, wie beispielsweise Silikonkleber, verklebt. Die Befestigungselemente können vollständig aus dem Klebstoff gebildet sein. Insbesondere können Fußenden der Befestigungselemente mit dem Leitungselement stoffschlüssig verbunden sein, wobei Kopfenden der Befestigungselemente aus den Durchtrittsöffnungen herausragen können, vorzugsweise sowohl radial nach außen wie auch seitlich über Außenränder der Durchtrittsöffnungen hinweg. Die Kopfenden können breiter als die Durchtrittsöffnungen ausgestaltet sein, so dass die Befestigungselemente eine formschlüssige Verbindung zwischen dem Leitungselement und Adapterelement bewirken, ähnlich einer Nietverbindung.

Bei einer Verwendung einer Kanülenanordnung oder einer Blutpumpenanordnung hier vorgeschlagener Art wird das Schutzelement beispielsweise gekürzt. Dies kann beispielsweise vor oder während einer Implantation der Kanülenanordnung bzw. der Blutpumpenanordnung durchgeführt werden. Hierbei werden zwei in der Abfolge benachbarte Segmente des Schutzelements voneinander getrennt. Dies kann beispielsweise erfolgen, indem mindestens ein, genau ein oder jedes Verbindungselement des Schutzelements, das die beiden benachbarten Segmente miteinander verbindet, mittels eines manuell geführten Schneidinstruments, beispielsweise mittels eines Skalpells, durchschnitten wird. Dies kann insbesondere im Fall der kabelförmigen Verbindungselemente besonders einfach durchgeführt werden. Zusätzlich oder alternativ, je nach Ausgestaltung der Verbindungselemente, können die beiden benachbarten Segmente auch dadurch getrennt werden, dass sie manuell auseinandergezogen werden, beispielsweise bis ein bestehender gegenseitiger Eingriff zwischen Verbindungselementen, die die beiden benachbarten Segmente miteinander verbinden, durch das Auseinanderziehen gelöst worden ist. Dies ist insbesondere möglich, wenn die Verbindungselemente, wie oben beschrieben, als komplementär ausgeformte axiale Endbereiche der Segmente ausgestaltet sind.

Prinzipiell kann die hier beschriebene Kanülenanordnung anstelle des hier vorgeschlagenen Schutzelements auch ein alternatives Schutzelement für die Kanüle aufweisen, das einen Führungskanal für die Kanüle, in dem die Kanüle ganz oder zumindest abschnittsweise verläuft, ausbildet und das beispielsweise in Form eines flexiblen Schlauches ausgestaltet sein kann. Auch in einer Kanülenanordnung mit einem solchen alternativen Schutzelement kann beispielsweise das hier beschriebene Anschlusselement der Kanüle (beispielsweise in Form einer Schelle) vorgesehen sein. Entsprechend kann die hier beschriebene Blutpumpenanordnung prinzipiell auch mit dieser alternativen Kanülenanordnung realisiert sein. Das alternative Schutzelement kann dann auf die gleiche Weise mit dem Konnektor verbunden sein wie das hier vorgeschlagene Schutzelement.

Die Kanülenanordnung ist typischerweise vollständig implantierbar. Hierzu kann neben der Kanüle insbesondere auch das Schutzelement, wie auch alle anderen Komponenten der Kanülenanordnung, ganz oder zumindest teilweise, insbesondere auf ihren Oberflächen, aus biokompatiblen Materialien bestehen.

Im Folgenden werden spezielle Ausführungsbeispiele der hier vorgeschlagenen Kanülenanordnung und der hier vorgeschlagenen Blutpumpenanordnung wie auch der Verwendung derselben anhand der in den Figuren 1 bis 7 gezeigten schematischen Darstellungen beschrieben. Es zeigen:
- Figur 1: eine seitliche Ansicht eines Beispiels einer Blutpumpenanordnung mit einer Kanülenanordnung hier vorgeschlagener Art,
- Figur 2: eine seitliche Ansicht der in Figur 1 gezeigten Kanülenanordnung,
- Figur 3: eine Schnittdarstellung der in Figur 2 gezeigten Kanülenanordnung gemäß dem in Figur 2 markierten und mit der Ziffer III bezeichneten Längsschnitt,
- Fign. 4A-4C: perspektivische Ansichten eines Beispiels für ein Schutzelement der in den Figuren 1 bis 3 gezeigten Kanülenanordnung,
- Figur 5A: eine Schnittdarstellung eines weiteren Beispiels für ein Schutzelement der in den Figuren 1 bis 3 gezeigten Kanülenanordnung gemäß einem Längsschnitt durch das Schutzelement entlang einer zentralen Längsachse,
- Figur 5B: eine perspektivische Ansicht eines einzelnen Segments des in Figur 5A gezeigten Schutzelements,
- Figur 5C: eine perspektivische Ansicht einer Abfolge von vier ineinandergesteckten Segmenten des in den Figuren 5A und 5B gezeigten Schutzelements,
- Figur 6: eine perspektivische Detailansicht eines vorderen Endes der in den Figuren 1 bis 3 gezeigten Kanülenanordnung und
- Figur 7: eine seitliche Ansicht mehrerer Komponenten der in Figur 1 gezeigte Blutpumpennordnung.

In den Figuren und der nachfolgenden Beschreibung sind identische oder einander entsprechende Merkmale jeweils mit den gleichen Bezugszeichen versehen.

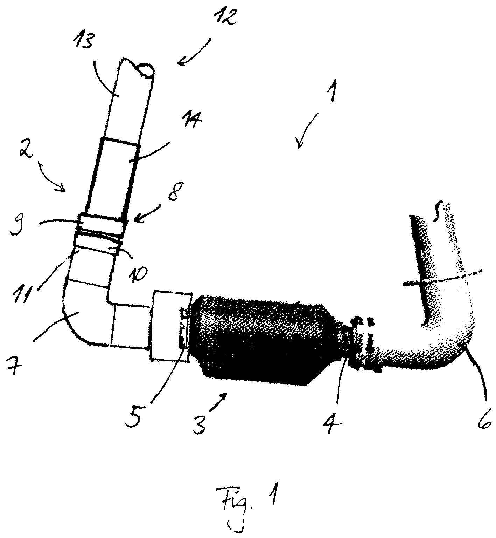

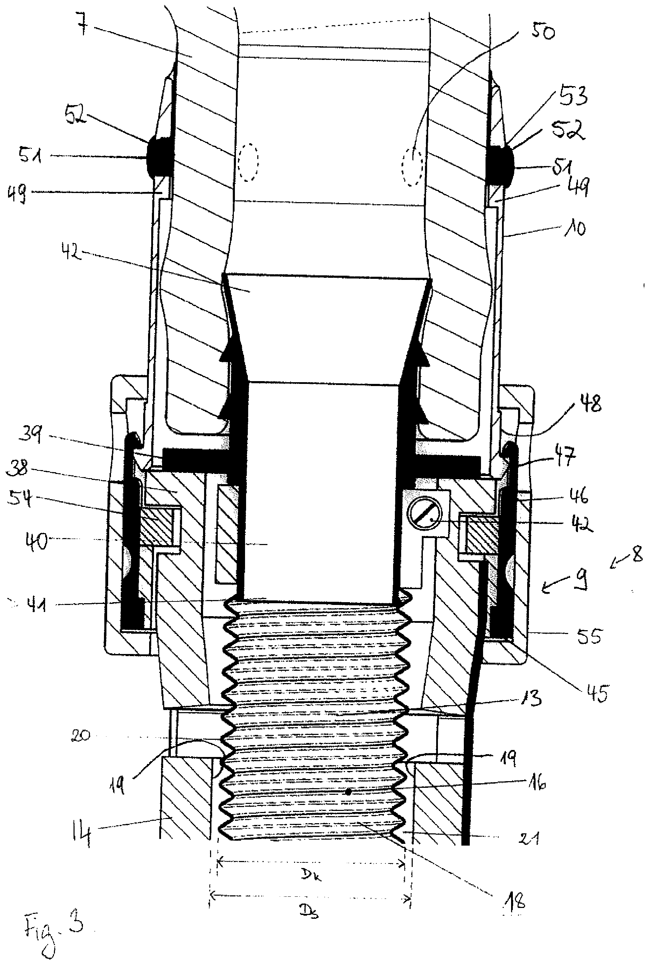

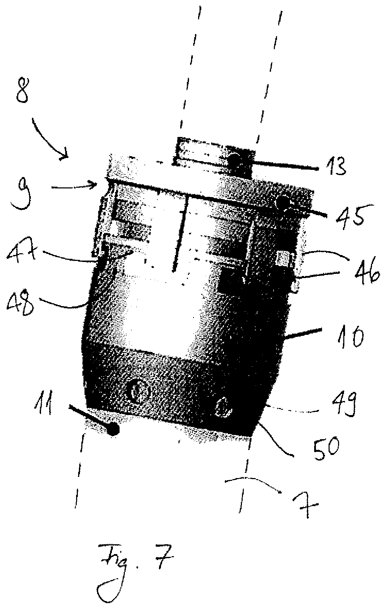

Figur 1 zeigt eine implantierbare Blutpumpenanordnung 1 hier vorgeschlagener Art, die eine implantierbare Kanülenanordnung 2 hier vorgeschlagener Art und eine implantierbare Blutpumpe 3 umfasst. Die Blutpumpe 3 ist vorliegend beispielsweise als eine Axialpumpe ausgestaltet und umfasst einen Einlass 4 und einen Auslass 5. Der Einlass 4 ist über einen Einlasskrümmer 6 der Blutpumpe 3 mit einer linken Herzkammer eines Herzens verbindbar. Der Auslass 5 ist über einen Auslasskrümmer 7 der Blutpumpe 3 mit einem vorderen Ende 8 der Kanülenanordnung 2 lösbar verbunden. Zu diesem Zweck umfasst die Kanülenanordnung 2 an ihrem vorderen Ende 8 einen Konnektor 9, der in diesem Beispiel als ein Schnappverbinder mit mehreren flexiblen Schnappelementen 46 ausgestaltet ist, siehe Figuren 3 und 7. Die Blutpumpe 3 umfasst ein mit dem Konnektor 9 korrespondierendes Verbindungsteil 10, welches an einem axialen Endbereich 11 des Auslasskrümmers 2 befestigt ist. Mit einem hinteren Ende 12 ist die Kanülenanordnung 2 beispielsweise mit einem Aortenbogen verbindbar.

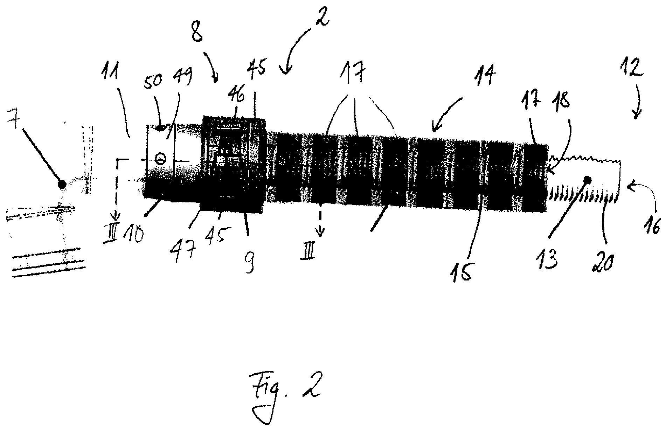

Die Kanülenanordnung 2 umfasst eine Kanüle 13 in Form eines Grafts, der beispielsweise eine wellschlauchförmige textile Trägerstruktur aus gewebten oder gestrickten Polyesterfasern aufweist, siehe auch Figuren 2 und 3. Die Trägerstruktur ist mit Dichtungsmaterial, beispielsweise mit Rindergelatine, abgedichtet und somit flüssigkeitsdicht. Die Kanüle 13 kann mit dem hinteren axialen Endstück der Kanüle direkt oder über ein Anschlusselement, beispielsweise über einen Nahtring (nicht dargestellt), mit dem Aortenbogen oder einem anderen Blutgefäß verbunden werden.

Die Kanülenanordnung 2 umfasst außerdem ein Schutzelement 14 für die Kanüle 2, welches möglichst verhindern soll, dass die relativ weiche und formflexible Kanüle 2 geknickt, zusammengedrückt oder beschädigt wird. Das Schutzelement 14 ist ein von der Kanüle 13 separates und unabhängig hergestelltes Bauteil der Kanülenanordnung 2. Wie besonders gut aus den Figuren 2 und 3 ersichtlich ist, bildet das Schutzelement 14 in seinem Innern einen Kanal 15 für die Kanüle 13. Die Kanüle 13 verläuft abschnittsweise durch diesen Kanal 15 hindurch. Entsprechend verläuft auch der durch die Kanüle 13 seitlich umgrenzte Fließkanal 16 abschnittsweise innerhalb des Kanals 15 des Schutzelements 14. Der Kanal 15 und der Fließkanal 16 verlaufen, zumindest in den Abschnitten, in denen der Fließkanal 16 durch den Kanal 14 verläuft, im Wesentlichen parallel und koaxial zueinander.

Beim Zusammenbau der Kanülenanordnung 2 wird die Kanüle 13 abschnittsweise in den Kanal 15 des Schutzelements 14 eingebracht, beispielsweise indem die Kanüle 13 in den Kanal 15 hineingezogen wird oder indem das Schutzelement 14 über die Kanüle 13 geschoben oder gezogen wird. Der Kanal 15 ist ausreichend breit, um axiale Verschiebungen des Schutzelements 14 relativ zur Kanüle 13 zu ermöglichen. Zu diesem Zweck ist ein Durchmesser D_{S} des im Wesentlichen kreisrunden Querschnitts des Kanals 15 größer als ein größter Außendurchmesser D_{K} der wellschlauchförmigen Kanüle 13.

Wie ebenfalls in den Figuren 2 und 3 gut zu erkennen ist, umfasst das Schutzelement 14 eine Mehrzahl von Segmenten 17, beispielsweise zehn Segmente 17. Die Segmente 17 sind in einer reihenförmigen Abfolge nebeneinander angeordnet. Jedes der Segmente 17 definiert einen axialen Teilabschnitt des Kanals 15 des Schutzelements 14. Zu diesem Zweck sind die Segmente 17 hülsenförmig ausgeformt und weisen jeweils eine Durchtrittsöffnung 18 auf, welche das jeweilige Segment 17 vollständig in Richtung der Abfolge der Segmente 17 durchlaufen und jeweils den genannten axialen Teilabschnitt des Kanals 15 bilden. Der oben beschriebene Durchmesser D_{K} entspricht daher dem Innendurchmesser der Segmente 17, das heißt dem Durchmesser der Durchtrittsöffnungen 18. Somit besteht zwischen Innenwänden 19 der Segmente 17, die den Kanal 15 jeweils radial umgrenzen und die Durchtrittsöffnungen 18 definieren, und einer äußeren Oberfläche 20 der Kanüle 13 ein freier Zwischenspalt 21, der zumindest abschnittsweise in Form eines Ringspalts ausgestaltet sein kann.

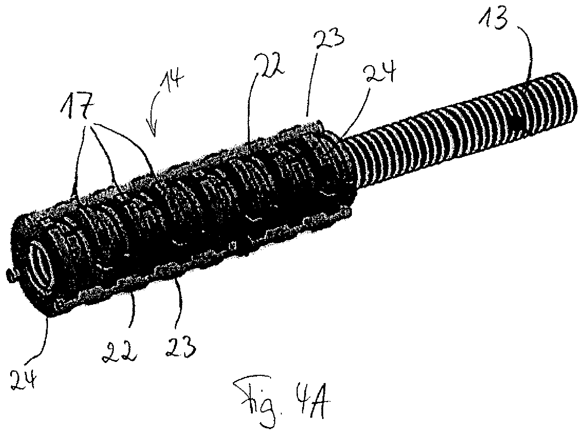

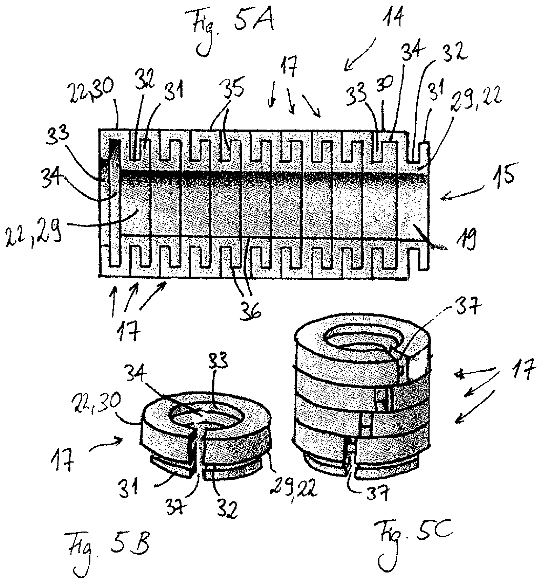

Das vorgeschlagene Schutzelement 14 umfasst außerdem Verbindungselemente 22, siehe Figuren 4A-C und Figuren 5A-5C, welche die Segmente 17 miteinander verbinden. Anhand der Figuren 4A-C und 5A-C werden weiter unten verschiedene konkrete Ausführungsbeispiele für derartige Verbindungselemente 22 detailliert beschrieben. Aufgrund der Verbindungselemente 22 bilden die Segmente 17 und die Verbindungselemente 22 des Schutzelements 14 eine Einheit, die unabhängig von der Kanüle 13 und anderen Bauteilen mechanisch stabil zusammenhält.

Die Segmente 17 sind ebenso wie die Verbindungselemente 22 vollständig aus einem elastisch verformbaren Polymer gebildet, beispielsweise aus Silikon. Die Segmente 17 wie auch die die Verbindungselemente 22 sind beispielsweise Spritzgussteile, also durch Spritzgießen hergestellt worden. Die Segmente 17 sind in einem mechanisch unbelasteten Zustand formidentisch; ebenso sind die Verbindungselemente 22 in einem mechanisch unbelasteten Zustand formidentisch.

Figuren 4A-4C zeigen ein spezielles Ausführungsbeispiel für das Schutzelement 14 der in Figuren 1-3 gezeigten Kanülenanordnung 2. Die Verbindungselemente 22 des Schutzelements 14 sind in diesem Beispiel als kabelförmige Elemente 23 ausgestaltet. Jedes der kabelförmigen Elemente 23 ist mit jedem der Segmente 17 direkt verbunden und an diesen direkt befestigt, beispielsweise durch Verkleben, beispielsweise mittels eines Silikonklebers. Die kabelförmigen Elemente 23 erstrecken sich jeweils in einer axialen Richtung entlang der Anordnung der Segmente 17, insbesondere also entlang des Kanals 15 des Schutzelements 14. Die kabelförmigen Elemente 23 sind schlauchartig geformt, könnten prinzipiell aber auch ohne einen inneren Hohlraum, also beispielsweise drahtförmig, ausgestaltet sein. Alternativ ist ein bandförmiges Element einsetzbar, welches beispielsweise eine Breite von einem Segmentumfang von 90° des Segmentes aufweist. Alternativ kann ein schlauchförmiges, beispielsweise perforiertes Element vorhanden sein, welches die Segmente schlauchartig umschließt. Die Perforation stellt hierbei eine Möglichkeit zur Gewährleistung der Verdrehbarkeit dar.

Die kabelförmigen Elemente 23 sind bezüglich einer azimutalen Umfangsrichtung um die Segmente 14 herum voneinander beabstandet angeordnet. Dabei sind die kabelförmigen Elemente 23 gleichmäßig über den Umfang der Segmente 17 verteilt. Im vorliegenden Beispiel von drei kabelförmigen Elementen 23 betragen die azimutalen Abstände zwischen ihnen etwa 120°.

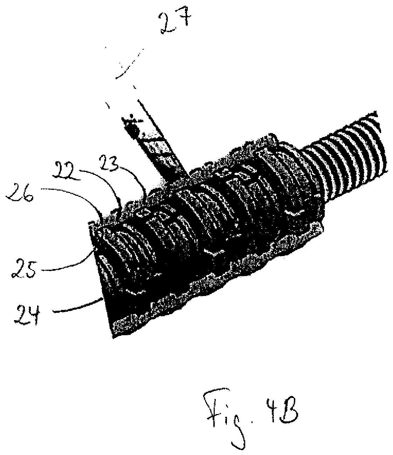

Jedes der Segmente 17 weist einen radial nach außen gerichteten Vorsprung 24 auf. An diesen Vorsprüngen 24 sind die kabelförmigen Elemente 23 abgestützt und befestigt, beispielsweise durch Kleben. Die Vorsprünge 24 sind als ringförmige Verbreiterungen der jeweiligen Segmente 17 ausgestaltet und weisen Aufnahmen 25 in Form radialer Vertiefungen auf, durch die jeweils ein axialer Teilabschnitt 26 eines kabelförmigen Elements 23 hindurch verläuft. Eine axiale Ausdehnung eines jeden der Vorsprünge 24 ist geringer als eine axiale Ausdehnung des jeweiligen Segments 17, so dass die kabelförmigen Elemente 23 zwischen den Vorsprüngen 24 in radialer Richtung zu den Segmenten 17 beabstandet sind, also freitragend sind. Somit können die kabelförmigen Elemente 23 in diesen Bereichen relativ leicht mittels eines handgeführten Schneidinstruments 27, beispielsweise mittels eines Skalpells, durchtrennt werden, ohne dabei das jeweilige Segment 17 oder gar die Kanüle 13 zu beschädigen, siehe Figur 4B.

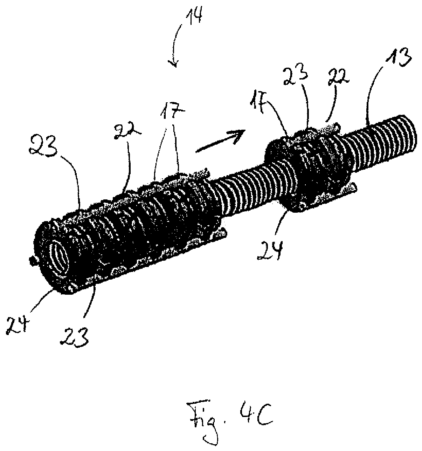

Wie in Figur 4C gezeigt ist, können nach dem Durchtrennen aller drei kabeiförmigen Elemente 23 zwischen zwei benachbarten Segmenten 17 die beiden getrennten Segmente 17 auseinandergezogen werden. Auf diese Weise können nicht benötigte Segmente 17 von der Kanülenanordnung 2 entfernt werden, indem sie beispielsweise über ein Ende der Kanüle 13 hinweg gezogen werden. Die Kanüle 13 kann, zuvor oder nachfolgend, ebenfalls mittels eines geeigneten Schneidinstruments gekürzt werden.

Außerdem ist es möglich, in der Abfolge benachbarte Segmente 17 relativ zueinander um eine Längsachse des Kanals 15 um 5° zu drehen, ohne die kabelförmigen Elemente 23, die Segmente 17 oder gar den Graft hierbei zu beschädigen.

Die Figuren 5A-5C zeigen ein weiteres spezielles Ausführungsbeispiel für das Schutzelement 14 der in den Figuren 1-3 gezeigten Kanülenanordnung 2. Die Verbindungselemente 22 des Schutzelements 14 sind in diesem Beispiel in axialen Endbereichen der Segmente 17 integriert. Jedes der Segmente 17 weist einen ersten axialen Endbereich 29 und einen zweiten axialen Endbereich 30 auf, wobei die ersten axialen Endbereiche 29 komplementär zu den zweiten axialen Endbereichen 30 geformt sind. Die ersten und die zweiten axialen Endbereiche 29, 30 von jeweils zwei in der Abfolge benachbarten Segmenten 17 überlappen sich axial und befinden sich in einem gegenseitigen Eingriff. Durch diesen Eingriff besteht eine mechanische Verbindung zwischen den Segmenten 17. Dadurch, dass die Segmente 17 in diesem Beispiel vollständig aus Silikon bestehen, sind die Segmente 17 als Ganzes und insbesondere auch in ihren axialen Endbereichen 29, 30 elastisch verformbar. Die formschlüssigen Verbindungen zwischen benachbarten Segmenten lassen sich daher manuell durch axiales Zusammenschieben oder Auseinanderziehen der Segmente 17 besonders einfach herstellen bzw. lösen.

Der erste axiale Endbereich 29 eines jeden Segments 17 weist einen ersten Steg 31 sowie eine erste Nut 32 auf, wobei der erste Steg 31 und die erste Nut 32 auf einer dem Kanal 15 radial abgewandten Außenseite 35 (also auf einer äußeren Oberfläche) des jeweiligen Segments 17 angeordnet sind. Der zweite axiale Endbereich 30 eines jeden Segments 17 weist einen zweiten Steg 33, der zur ersten Nut 32 komplementär geformt ist, sowie eine zweite Nut 34, die zum ersten Steg 31 komplementär geformt ist, auf, wobei der zweite Steg 33 und die zweite Nut 34 auf einer dem Kanal 15 radial zugewandten Innenseite 36 (also auf einer inneren Oberfläche) des jeweiligen Segments 17 angeordnet sind. Der erste Steg 31 grenzt axial an die erste Nut 32 an. Entsprechend grenzt der zweite Steg 33 axial an die zweite Nut 34 an. Bei dem gegenseitigen Eingriff des ersten axialen Endbereichs 29 mit dem zweiten axialen Endbereich zweier in der Abfolge benachbarter Segmente 17 hintergreifen sich die ersten und zweiten Stege 31, 33 gegenseitig axial, wobei der erste Steg 31 in die zweite Nut 34 und der zweite Steg 33 in die erste Nut 34 eintaucht. Die ersten Stege 31 und die zweiten Nuten 34 sind radial nach außen gerichtet. Die zweiten Stege 33 und die ersten Nuten 32 sind radial nach innen gerichtet. Die ersten und zweiten Stege 31, 33 und die ersten und zweiten Nuten 32, 34 umlaufen das jeweilige Segment in einer Umfangsrichtung (azimutal) vollständig. Es ist möglich, in der Abfolge benachbarte Segmente 17 relativ zueinander um eine Längsachse des Kanals 15 um 360° zu drehen, ohne die Segmente 17 voneinander zu lösen oder den Graft zu beschädigen.

Wie in den Figuren 5B und 5C erkennbar ist, weisen die Segmente 17 jeweils einen Schlitz 37 auf, der sich in axialer Richtung über eine axiale Gesamtlänge des jeweiligen Segments 17 und in radialer Richtung von der Außenseite 35 (äußere Oberfläche) des jeweiligen Segments 17 bis zur Innenseite 36 (innere Oberfläche), also bis zum Kanal 15 hin erstreckt. Der Schlitz 37 eines jeden Segments 17 kann manuell soweit aufgeweitet werden, bis das Segment 17, sofern es zuvor von den benachbarten Segmenten 17 getrennt worden ist, von der Kanüle 13 in einer seitlichen, nichtaxialen Richtung heruntergezogen werden kann. Auch die in den Figuren 4A-4C dargestellten Segmente können jeweils einen derartigen Schlitz 37 aufweisen.

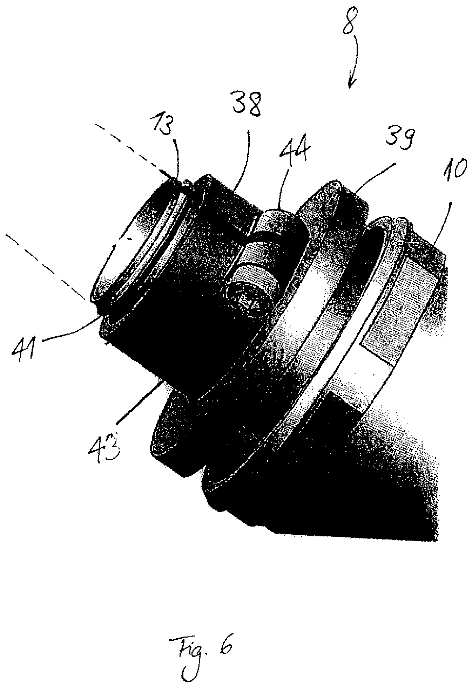

Wie beispielsweise aus den Figuren 3 und 6 ersichtlich ist, weist die Kanüle 13 an dem vorderen Ende 8 der Kanülenanordnung 2 ein Anschlusselement 38 auf, das dazu ausgestaltet ist, eine fluiddichte Verbindung zwischen der Kanüle 13 und einem weiteren Leitungselement 39 für die zu leitende Flüssigkeit herzustellen. Bei dem Leitungselement 39 handelt es sich in diesem Beispiel um ein Rohrelement (Olive) aus Titan, das mit einem ersten Ende 40 (Rohrstutzen) in einen vorderen axialen Endbereich 41 der Kanüle 2 eingeschoben ist. Ein zweites Ende 42 (Rohrstutzen) des Leitungselements 39 ist in den axialen Endbereich 11 des aus Silikon gefertigten Auslasskrümmers 7 eingeschoben.

Das Anschlusselement 38 ist in Form einer metallischen Schelle ausgestaltet, welche den axialen Endbereich 41 der Kanüle 13 umgreift. Die Schelle kann mittels einer Spannschraube 43 und eines korrespondierenden Gewindeelements 44 gespannt werden, um den axialen Endbereich 41 der Kanüle radial nach innen auf das erste Ende 40 des Leitungselements 39 aufzupressen und so eine fluiddichte Klemmverbindung zwischen der Kanüle 2 und dem Leitungselement 39 herzustellen.

Wie am besten in den Figuren 3 und 7 zu erkennen ist, ist der Konnektor 9 am vorderen Ende 8 der Kanülenanordnung 2 an einem vordersten Segment 17 des Schutzelements 14 befestigt. Der als Schnappverbinder ausgestaltete Konnektor 9 umfasst einen Klauenring 46, der mehrere als Klauen ausgestaltete flexible Schnappelemente 46 aufweist. Der Klauenring 45 kann beispielsweise in der Art des in der Veröffentlichung WO 2004/001272 A1 beschriebenen Klauenrings ausgestaltet sein. Der Klauenring 45 ist vorzugsweise drehbar und/oder (axial) verschiebbar auf einem vordersten Segment 17 des Schutzelements 14 montiert. Der Konnektor 9 umfasst außerdem einen Anschlagring 54. Der Klauenring 46 drückt im eingerasteten Zustand mit einer axialen Anpresskraft auf den Anschlagring 54, welcher die axiale Anpresskraft auf das vorderste Segment 17 überträgt. Beispielsweise wird auf diese Weise eine axiale Stirnfläche des vordersten Segments 17 beispielsweise auf eine angrenzende axiale Stirnfläche des Leitungselements 39 oder des Verbindungsteils angepresst.

Das korrespondierende Verbindungsteil 10 weist Halteflächen 47 zum Herstellen einer lösbaren Rastverbindung zwischen den Halteflächen 47 und den Schnappelementen 46 (Klauen) auf. Das Verbindungsteil 10 umfasst einen Rastring 48, auf dem die Halteflächen als Rastringhafteflächen ausgebildet sind. Der Rastring 48 und/oder die Halteflächen 48 können beispielsweise in der Art des in der Veröffentlichung WO 2004/001272 A1 beschriebenen Rastrings bzw. der dort beschriebenen Rastringhalteflächen ausgestaltet sein.

Das Verbindungsteil 10 ist ein Adapterelement, das ganz oder zumindest teilweise aus Titan gefertigt ist und das mit dem Auslasskrümmer 7 der Blutpumpe 3 mittels eines Klebstoffs fest verbunden ist. Als Klebstoff kommt beispielsweise ein Silikonkleber in Frage. Das Adapterelement kann einen hülsenförmigen Bereich 49 aufweisen, der den axialen Endbereich 11 des Auslasskrümmers 7 ringsum vollständig umschließt. Im gezeigten Beispiel ist der Rastring 48 in das Verbindungsteil 10 integriert und grenzt axial an den hülsenförmigen Bereich an, ist also als ein axialer Endbereich des Verbindungsteils 10 ausgestaltet. Eine Schutzhülle 55 bildet eine äußere Hülle um den Klauenring 46 und den Rastring 48.

Der hülsenförmige Bereich 49 weist radial verlaufende Durchtrittsöffnungen 50 auf. Ausgehend von dem axialen Endbereich 11 verlaufen Befestigungselemente 51 durch die Durchtrittsöffnungen 50 hindurch, welche das Adapterelement mit dem Auslasskrümmer 11 verbinden. Die Befestigungselemente 51 bestehen aus dem oben genannten Klebstoff und sind mit ihren Fußenden 52 mit dem axialen Endbereich 11 des Auslasskrümmers 7 stoffschlüssig verbunden (verklebt). Kopfenden 53 der Befestigungselemente 51 ragen aus den Durchtrittsöffnungen 51 heraus und ragen dabei sowohl radial nach außen wie auch seitlich über die Außenränder der Durchtrittsöffnungen 51 hinaus. Da die Kopfenden 53 breiter als die Durchtrittsöffnungen 50 ausgestaltet sind, bewirken die Befestigungselemente 51 eine formschlüssige Verbindung zwischen dem Auslasskrümmer 7 und dem Adapterelement, ähnlich einer Nietverbindung.

### Bezugszeichenliste:

- 1: Blutpumpenanordnung
- 2: Kanülenanordnung
- 3: Blutpumpe
- 4: Einlass
- 5: Auslass
- 6: Einlasskrümmer
- 7: Auslasskrümmer
- 8: vorderes Ende
- 9: Konnektor
- 10: Verbindungsteil
- 11: axialer Endbereich
- 12: hinteres Ende
- 13: Kanüle
- 14: Schutzelement
- 15: Kanal
- 16: Fließkanal
- 17: Segment
- 18: Durchtrittsöffnung
- 19: Innenwand
- 20: äußere Oberfläche
- 21: Zwischenspalt
- 22: Verbindungselement
- 23: kabelförmiges Element
- 24: Vorsprung
- 25: Aufnahmen
- 26: Teilabschnitt
- 27: Schneidinstrument
- 29: erster axialer Endbereich
- 30: zweiter axialer Endbereich
- 31: erster Steg
- 32: erste Nut
- 33: zweiter Steg
- 34: zweite Nut
- 35: Außenseite
- 36: Innenseite
- 37: Schlitz
- 38: Anschlusselement
- 39: Leitungselement
- 40: erstes Ende
- 41: axialer Endbereich
- 42: zweites Ende
- 43: Spannschraube
- 44: Gewindeelement
- 45: Klauenring
- 46: Schnappelement, Klaue
- 47: Haltefläche
- 48: Rastring
- 49: Bereich
- 50: Durchtrittsöffnung
- 51: Befestigungselement
- 52: Fußende
- 53: Kopfende
- 54: Anschlagring
- 55: Schutzhülle

## Patentansprüche

1. Kanülenanordnung (2), umfassend eine Kanüle (13), insbesondere einen Graft, zur Bildung eines Fließkanals (16) für Körperflüssigkeiten, insbesondere für Blut, und ein Schutzelement (14) für die Kanüle (13), wobei das Schutzelement (14) einen Kanal (15) für die Kanüle (13) definiert, wobei die Kanüle (13) zumindest abschnittsweise durch den Kanal (15) des Schutzelements (14) hindurch verläuft., wobei das Schutzelement (14) eine Mehrzahl von Segmenten (17) umfasst, wobei die Segmente (17) in einer Abfolge nebeneinander angeordnet sind und wobei jedes der Segmente (17) einen Teilabschnitt des Kanals (15) des Schutzelements (14) definiert, wobei die Segmente (17) derart miteinander verbunden sind, dass die Segmente (17) relativ zueinander um eine Längsachse des Kanals (15) drehbar sind.

2. Kanülenanordnung (2) nach Anspruch 1, **dadurch gekennzeichnet, dass** die Kanüle (13) ein Graft ist, der eine textile Trägerstruktur aufweist.

3. Kanülenanordnung (2) nach einem der vorangehenden Ansprüche,
**dadurch gekennzeichnet, dass** die Segmente (17) derart miteinander verbunden sind, dass in der Abfolge benachbarte Segmente (17) relativ zueinander um eine Längsachse des Kanals (15) um mindestens 3° oder um mindestens 5° drehbar sind.

4. Kanülenanordnung (2) nach einem der vorangehenden Ansprüche,
**dadurch gekennzeichnet, dass** ein Innendurchmesser (D_{S}) der Segmente (17) größer als ein Außendurchmesser (D_{K}) der Kanüle (13) ist.

5. Kanülenanordnung (2) nach einem der vorangehenden Ansprüche,
**dadurch gekennzeichnet, dass** die Segmente (17) ganz oder zumindest teilweise aus einem elastisch verformbaren Polymer gebildet sind, beispielsweise aus einem Silikon, einem Silikonelastomer oder einem Polyurethan.

6. Kanülenanordnung (2) nach einem der vorangehenden Ansprüche,
**dadurch gekennzeichnet, dass** die Segmente (17) Spritzgussteile sind.

7. Kanülenanordnung (2) nach einem der vorangehenden Ansprüche,
**dadurch gekennzeichnet, dass** das Schutzelement (14) mindestens ein kabelförmiges Element (23) umfasst, wobei das mindestens eine kabelförmige Element (23) mit zwei oder mit mehr als zwei der Segmente (17) oder mit jedem der Segmente (17) direkt verbunden ist.

8. Kanülenanordnung (2) nach Anspruch 7, **dadurch gekennzeichnet, dass** das mindestens eine kabelförmige Element (23) zwei oder mehr derartige kabelförmige Elemente (23) umfasst, wobei die kabelförmigen Elemente (23) in einer Umfangsrichtung um das Schutzelement (14) voneinander beabstandet angeordnet sind.

9. Kanülenanordnung (2) nach einem der Ansprüche 7 oder 8, **dadurch gekennzeichnet, dass** die Segmente (17) radial nach außen gerichtete Vorsprünge (24) aufweisen, an denen das mindestens eine kabelförmige Element (23) abgestützt und/oder befestigt ist, wobei das mindestens eine kabelförmige Element (23) zwischen den radial nach außen gerichteten Vorsprüngen (24) ganz oder zumindest abschnittsweise freitragend verläuft.

10. Kanülenanordnung (2) nach einem der vorangehenden Ansprüche,
**dadurch gekennzeichnet, dass** jedes der Segmente (17) einen ersten axialen Endbereich (29) und einen zweiten axialen Endbereich (30) aufweist, wobei die ersten axialen Endbereiche (29) der Segmente (17) komplementär zu den zweiten axialen Endbereichen (30) der Segmente (17) geformt sind, wobei sich die ersten und zweiten axialen Endbereiche (29, 30) von Segmenten (17), die in der Abfolge benachbart sind, axial überlappen und sich in einem gegenseitigen Eingriff befinden.

11. Kanülenanordnung (2) nach Anspruch 10, **dadurch gekennzeichnet, dass** der erste axiale Endbereich (29) eines jeden Segments (17) einen ersten Steg (31) sowie eine erste Nut (32) aufweist, wobei der erste Steg (31) und die erste Nut (32) auf einer dem Kanal (15) abgewandten Außenseite (35) des jeweiligen Segments (17) angeordnet sind, wobei der zweite axiale Endbereich (30) eines jeden Segments (17) einen zweiten Steg (33), der zur ersten Nut (32) komplementär geformt ist, sowie eine zweite Nut (34), die zum ersten Steg (31) komplementär geformt ist, aufweist, wobei der zweite Steg (33) und die zweite Nut (34) auf einer dem Kanal (15) zugewandten Innenseite (36) des jeweiligen Segments (17) angeordnet sind.

12. Kanülenanordnung (2) nach einem der vorangehenden Ansprüche,
**dadurch gekennzeichnet, dass** eines, mehrere oder jedes der Segmente (17) jeweils einen Schlitz (37) aufweist bzw. aufweisen, der sich in axialer Richtung über eine axiale Gesamtlänge des jeweiligen Segments (17) und in radialer Richtung von einer dem Kanal (15) abgewandten Außenseite (35) des jeweiligen Segments (17) bis zu einer dem Kanal (36) zugewandten Innenseite des Segments (17) hin erstreckt.

13. Kanülenanordnung (2) nach Anspruch 12, **dadurch gekennzeichnet, dass** die Kanüle (13) ein Anschlusselement (38) in Form einer Schelle aufweist, welche einen axialen Endbereich (41) der Kanüle (13) umgreift.

14. Kanülenanordnung (2), umfassend eine Kanüle (13), insbesondere einen Graft, zur Bildung eines Fließkanals (16) für Körperflüssigkeiten, insbesondere für Blut, und ein Schutzelement (14) für die Kanüle (13), wobei das Schutzelement (14) einen Kanal (15) für die Kanüle (13) definiert, wobei die Kanüle (13) zumindest abschnittsweise durch den Kanal (15) des Schutzelements (14) hindurch verläuft, wobei das Schutzelement (14) eine Mehrzahl von Segmenten (17) umfasst, wobei die Segmente (17) in einer Abfolge nebeneinander angeordnet sind und wobei jedes der Segmente (17) einen Teilabschnitt des Kanals (15) des Schutzelements (14) definiert, wobei die Schutzelemente formschlüssig miteinander verbunden sind.

15. Kanülenanordnung (2), umfassend eine Kanüle (13), insbesondere einen Graft, zur Bildung eines Fließkanals (16) für Körperflüssigkeiten, insbesondere für Blut, und ein Schutzelement (14) für die Kanüle (13), wobei das Schutzelement (14) einen Kanal (15) für die Kanüle (13) definiert, wobei die Kanüle (13) zumindest abschnittsweise durch den Kanal (15) des Schutzelements (14) hindurch verläuft, wobei das Schutzelement (14) eine Mehrzahl von Segmenten (17) umfasst, wobei die Segmente (17) in einer Abfolge nebeneinander angeordnet sind und wobei jedes der Segmente (17) einen Teilabschnitt des Kanals (15) des Schutzelements (14) definiert, wobei das Schutzelement (14) Verbindungselemente (22) umfasst, welche die Segmente (17) miteinander verbinden, wobei die Verbindungselemente (22) stoffschlüssig mit den Segmenten (17) verbunden sind.

16. Blutpumpenanordnung (1) mit einer Kanülenanordnung (2) nach einem der vorangehenden Ansprüche und einer Blutpumpe (3), wobei die Kanülenanordnung (2) an einem vorderen Ende (8) der Kanülenanordnung (2) einen Konnektor (9) aufweist, wobei die Blutpumpe (3) ein mit dem Konnektor (9) korrespondierendes Verbindungsteil (10) zum Herstellen einer lösbaren Verbindung zwischen der Kanülenanordnung (2) und der Blutpumpe (3) aufweist.

17. Blutpumpenanordnung nach Anspruch 16, **dadurch gekennzeichnet, dass** der Konnektor (9) ein Schnappverbinder mit mindestens einem flexiblen Schnappelement (46) ist und dass das Verbindungsteil (10) der Blutpumpe (3) mindestens eine Haltefläche (47) zum Herstellen einer lösbaren Rastverbindung zwischen der mindestens einen Haltefläche (47) und dem mindestens einen Schnappelement (46) aufweist.

18. Blutpumpenanordnung (1) nach einem der Ansprüche 16 oder 17, **dadurch gekennzeichnet, dass** das Verbindungsteil (10) der Blutpumpe (3) ein Adapterelement ist, das mit einem Leitungselement der Blutpumpe fest verbunden ist, beispielsweise mittels eines Klebstoffs.

19. Blutpumpenanordnung (1) nach Anspruch 18, **dadurch gekennzeichnet, dass** das Adapterelement einen Bereich (49) aufweist, der ein Ende des Leitungselements zumindest teilweise umschließt, wobei der Bereich (49) im Wesentlichen radial verlaufende Durchtrittsöffnungen (50) aufweist, wobei ausgehend von dem Leitungselement Befestigungselemente (51) durch die Durchtrittsöffnungen (50) hindurch verlaufen, welche das Adapterelement mit dem Leitungselement verbinden.

20. Blutpumpenanordnung (1) nach einem der Ansprüche 18 oder 19, **dadurch gekennzeichnet, dass** das Adapterelement teilweise oder ganz aus einem metallischen Werkstoff gefertigt ist, vorzugsweise aus Titan.

21. Verwendung einer Kanütenanordnung (2) nach einem der Ansprüche 1 bis 15 oder einer Blutpumpenanordnung (1) nach einem der Ansprüche 16 bis 20, **dadurch gekennzeichnet, dass** zum Kürzen des Schutzelements (14) zwei in der Abfolge benachbarte Segmente (17) voneinander getrennt werden,
- indem mindestens ein oder ein jedes Verbindungselement (22) von Verbindungselementen (22) des Schutzelements (14), welches die beiden benachbarten Segmente (17) miteinander verbindet, mittels eines manuell geführten Schneidinstruments, beispielsweise mittels eines Skalpells, durchschnitten wird und/oder
- indem die beiden benachbarten Segmente (17) manuell auseinandergezogen werden, bis ein bestehender gegenseitiger Eingriff zwischen Verbindungselementen (22), welche die beiden benachbarten Segmente (17) miteinander verbinden, durch das Auseinanderziehen gelöst worden ist.
